# EUROPÄISCHE PATENTSCHRIFT

(11) **EP 0 581 935 B1**
(45) Veröffentlichungstag und Bekanntmachung des Hinweises auf die Patenterteilung: **02.05.1997**
(21) Anmeldenummer: 93903955.8
(22) Anmeldetag: 11.02.1993
(51) Int. Cl.: A61F 2/80

(54) **BEINPROTHESE**
LEG PROSTHESIS
PROTHESE DE JAMBE

(30) Priorität: 14.02.1992 DE 4204482
(43) Veröffentlichungstag der Anmeldung: 09.02.1994
(73) Patentinhaber: POHLIG, Kurt, 83278 Traunstein (DE)
(72) Erfinder: POHLIG, Kurt, 83278 Traunstein (DE)
(74) Vertreter: Bauer, Robert, Dipl.-Ing.
(86) Internationale Anmeldenummer: EP9300333
(87) Internationale Veröffentlichungsnummer: WO9315696

(56) Entgegenhaltungen:
- EP-A- 0 019 612
- EP-A- 0 151 834
- DE-A- 2 540 138
- FR-A- 1 548 465
- US-A- 1 893 853
- US-A- 3 671 980
- US-A- 4 923 475
- US-A- 5 108 456
- US-A- 5 139 523

## Beschreibung

Die Erfindung betrifft eine Beinprothese gemäß Gattungsbegriff des Anspruchs 1.

Vor allem bei Beinprothesen ist man im allgemeinen bestrebt, den Schaft möglichst genau den Konturen des Stumpfes anzupassen, um neben einem festen Sitz der Prothese eine möglichst großflächige Druckverteilung zu erreichen. Die Anpassung geht dabei neuerdings so weit, daß sich in dem Schaft infolge "Pumpens" beim Gehvorgang bewußt ein Unterdruck einstellt, durch den der Schaft, ggf. sogar ohne zusätzliche Hilfsmittel, am Stumpf gehalten wird. Zudem sucht man - neuerdings selbst bei Oberschenkelprothesen - die Stützkraft der Prothese, zumindest überwiegend, über den Stumpf und den Oberschenkelknochen (Femur) anstatt über das Sitzbein (Os ischii) in den Körper des Prothesenträgers einzuleiten. Untersuchungen haben indessen gezeigt, daß der feste Einschluß des Stumpfes in dessen Umfangsbereich ohne gleichzeitigen Stumpfendkontakt zu Stauerscheinungen mit Folgen bis hin zu einem chronischen ödem führen kann (vergl. Baumgartner und Botta "Amputation und Prothesenversorgung der unteren Extremität", Ferdinand Enke Verlag Stuttgart, 1989, S. 237-239).

Abgesehen davon ist es wünschenswert, die dem Stumpf vermittelte Stützkraft möglichst unmittelbar in den bzw. die darin enthaltenen Knochen und damit in das anschließende Gelenk (Hüft- bzw. Kniegelenk) einzuleiten. Man hat dazu, insbesondere durch anmodellierte Innenschäfte, eine möglichst vollkommene Einbettung des Stumpfes zu erzielen versucht, doch ist eine solche aufgrund der sich unvermeidlich einstellenden länger- wie auch kurzfristigen Formänderungen des Stumpfes etwa durch Muskelschwund oder stoffwechselbedingte An- und Abschwellungen allein durch diese Maßnahme nicht befriedigend zu erreichen.

Dies erkennend wurde denn etwa durch die US-PSen 1 893 853 und 2 634 424, das DE-GM 1 810 432 - wovon im Gattungsbegriff ausgegangen wird - und, mit einer zusätzlichen Einlage aus porösem plastischem Werkstoff, auch das DE-GM 1 882 630 bereits vorgeschlagen, in den Prothesenschaft ein ringsherumlaufendes, aufblasbares Kissen einzubringen, das sich den Volumenänderungen des Stumpfes an der nämlichen Stelle anpassen können soll, doch ist auch dadurch, selbstredend, ein Stumpfendkontakt nicht herzustellen. Aus der EP-OS 0 151 834 ist des weiteren zwar bereits eine zusammenfaltbare Unterschenkelprothese für den gelegentlichen Gebrauch etwa in Duschkabinen bekannt, bei der neben aufblasbaren Kissen im Stumpfumfangsbereich auch ein solches im Stumpfendbereich auftritt. Damit wird jedoch eine satte Einbettung des Stumpfes, wie sie für einen Stumpfendkontakt wesentlich ist, weder angestrebt noch erreicht.

Der Erfindung liegt von daher die Aufgabe zugrunde, eine Beinprothese gemäß Gattungsbegriff so auszubilden, daß eine weitestgehende satte Einbettung des Stumpfes wie auch die Aufbringung einer kontrollierten Stumpfendbealstung ermöglicht wird.

Diese Aufgabe ist erfindungsgemäß mit den Kennzeichnungsmerkmalen des Anspruchs 1 gelöst. Die Unteransprüche geben darüberhinausgehend vorteilhafte Ausgestaltungsmöglichkeiten an.

Das im Stumpfendbereich des im wesentlichen bereits formangepaßten Prothesenschaftes angeordnete, anschmiegsame aufblähbare Kissen ermöglicht es, unter Ausnutzung der durch die seitlichen Backen an dem Stumpf hervorrufbaren Flächenhaftung einen dauerhaften, schonenden Stumpfendkontakt mit kontrollierter überdurchschnittlicher Stumpfendbelastung herzustellen, um so zugleich den bzw. die maßgeblichen Knochen des Stumpfes in für Prothesenträger ganz ungewöhnlichem Maße an der Lastaufnahme und -weiterleitung zu beteiligen.

Von den Unteransprüchen kommt den Ansprüchen 2 und 3 besondere Bedeutung zu, indem eine flexible Innenschale und die Anordnung seitlicher Backen außerhalb derselben über die Flexibilität der Innenschale an den nämlichen Stellen eine besonders stetige Druckaufbringung auf den Stumpf ermöglicht, während andererseits die innenseitige Anordnung des endseitigen Kissens ungeachtet der dortigen formbedingten geringeren Flexibilität des Innenschaftes eine satte Anschmiegung an das Stumpfende zuläßt.

Sodann hat man noch erkannt, daß nicht alle Stellen im Stumpfumfangsbereich gleichermaßen für eine seitliche Druckaufnahme geeignet sind, und dem Erfinder blieb es vorbehalten, hierfür hinsichtlich Oberschenkelprothesen vor allem die im Anspruch 4 angegebenen Bereiche als prädestiniert zu erkennen. Zudem bietet die Druckaufbringung mittels der langgestreckten, im dorso-lateralen Bereich an der Kante des Oberschenkelknochens angeordneten Backe die Möglichkeit, im Sinne einer Korrekturpelotte die sonst bei Oberschenkelamputierten sich gewöhnlich einstellende Abduktions-Fehlstellung des Oberschenkelknochens zu korrigieren und so die für die Stabilisierung des Beckens notwendige natürliche Muskelvorspannung wiederherzustellen.

Nachfolgend werden einige Ausführungsbeispiele in Verbindung mit den Zeichnungen anhand einer Oberschenkelprothese genauer beschrieben. Dabei zeigt
- Fig. 1: einen Längsschnitt durch den Schaft einer Oberschenkelprothese der beanspruchten Art mit Innenschaft, etwas schematisiert,
- Fig. 2: einen Querschnitt durch den gleichen Schaft und Innenschaft in der Ebene der Linie II-II von Fig. 1,
- Fig. 3: ein Detail etwa nach Fig. 1 in vergrößerter Darstellung mit zusätzlichen Einzelheiten,
- Fig. 4: schematisch das Prinzip einer anderen Ausführungsform,
- Fig. 5: ein entsprechendes Schema einer weiteren Ausführungsform und
- Fig. 6: ein Detail aus einem Längsschnitt durch eine Prothese ohne Innenschaft mit einer anders ausgebildeten seitlichen Backe.

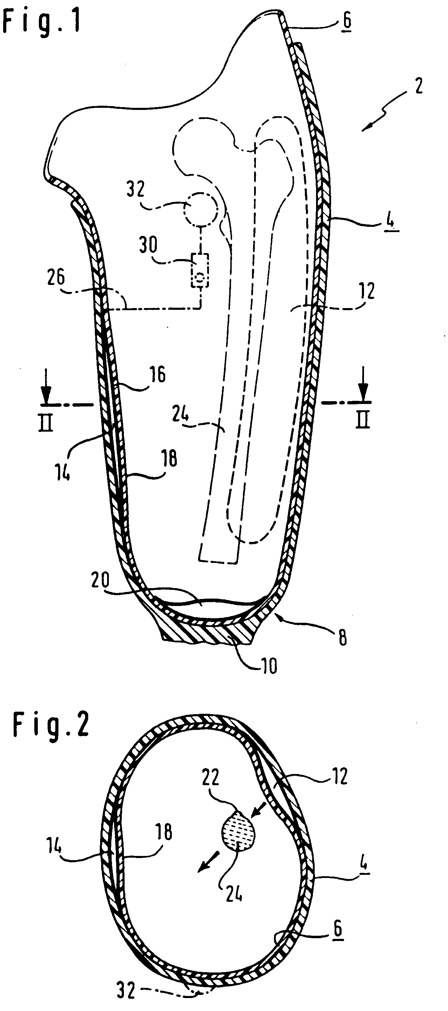

Die in Fig. 1 und 2 nur zum Teil dargestellte Oberschenkelprothese 2 weist in insoweit herkömmlicher Weise einen durch (nicht gezeigte) Armierungen oder dergl. verhältnismäßig steifen, schalenförmigen Außenschaft 4 und einen herausnehmbar darin eingesetzten, dem jeweiligen Stumpf individuell angepaßten, vergleichsweise flexiblen Innenschaft 6 auf. An das geschlossene distale Ende 8 des Außenschaftes 4 schließt sich ein zum Kniegelenk hin führendes säulenartiges Bauteil 10 an (Ummantelung in der Darstellung weggelassen).

Abweichend von herkömmlichen derartigen Prothesen sind nun im dargestellten Fall zwischen den längsverlaufenden Wandungen des Außenschaftes 4 und des Innenschaftes 6 zwei diskrete aufblähbare Kissen 12 und 14 angeordnet, die mit ihrer innenliegenden Wand, wie z.B. 16, Backen bilden, von denen die Wandung 18 des Innenschaftes 6 in den betreffenden Bereichen nach innen gedrückt werden kann. Ein weiteres aufblähbares Kissen, 20, befindet sich innenseitig im Innenschaft 6 an dessen distalem Ende, wo die Wandung 18 des Innenschaftes wegen der zweidimensionalen und zudem stärkeren Wölbung nicht gleichermaßen flexibel ist, und zumindest dieses Kissen besteht aus einem verhältnismäßig weichen, elastisch dehnfähigen Material, wie z.B. Gummi oder elastischem Polyurethan, kaschiert z.B. mit Textilien oder unkaschiert, um sich dem Stumpfende satt anschmiegen zu können.

Genauer gesagt sind die Kissen 12 und 14 im dorso-lateralen Bereich im Anschluß an die Kante 22 des (gestrichelt angedeuteten) Oberschenkelknochens (Femur) 24 bzw. im medial-distalen Bereich angeordnet. Dabei weist das Kissen 12, sich von proximal nach distal erstreckend, eine langgestreckte, das Kissen 14 hingegen eine eher runde Form auf.

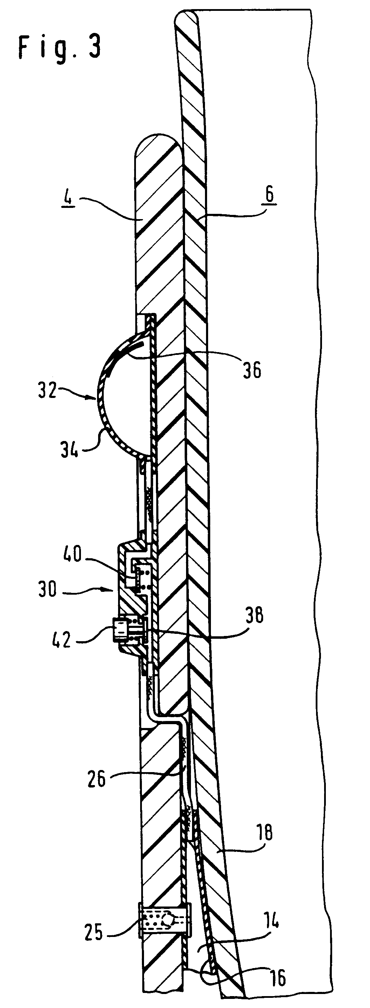

Wie durch die Pfeile in Fig. 2 angedeutet, erfährt der Oberschenkelknochen 24 durch das Kissen 12 in gewollter Weise eine mehr oder weniger starke Adduktion, womit, wie gesagt, die sonst sich bei Oberschenkelamputierten zumeist einstellende Abduktion korrigierbar ist. Daneben erlauben es die Kissen 12 und 14, das Schaftvolumen zu reduzieren und dem Stumpf im Schaft, genauer gesagt in dem Innenschaft 6, eine verstärkte Flächenhaftung zu vermitteln. Diese Flächenhaftung wiederum gestattet es, mit Hilfe des Kissens 20 einen satten Stumpfendkontakt mit dosierbarer Stumpfendbelastung herbeizuführen bzw. die Stumpfendbelastung in wünschenswerter Weise zu erhöhen. In diesem Zusammenhang kann gewünschtenfalls ein Druckbegrenzungsventil Anwendung finden, wie eines in Fig. 3 am Kissen 14 als überdruckventil 25 gezeigt ist. Im übrigen aber gestatten es die Kissen, den Innenschaft 6 so voluminös zu gestalten, daß sich bei entlüfteten Kissen das Anziehen der Prothese ganz wesentlich erleichtert.

Wie in Fig. 1 angedeutet und genauer aus Fig. 3 ersichtlich, führt von dem jeweiligen Kissen, wie z.B. 14, ein feiner Schlauch 26 zu einem an der Prothese 2 angebrachten - oder aber auch separaten - Ventil 30, an das sich eine kleine manuell betätigbare Luftpumpe 32 anschließt, wie sie im Prinzip etwa von Parfumzerstäubern her bekannt ist. Im gezeigten Beispiel besteht die Luftpumpe 32 im wesentlichen aus einer sich durch Eigenspannung erweiternden flachen Gummibirne 34, an der ein klappenartiges Rückschlagventil 36 als Lufteinlaßventil angeordnet ist. Die Luftpumpe 32 ist unaufällig unter der Kleidung zu tragen und im Bedarfsfall durch diese hindurch durch wiederholtes Zusammendrücken und Entspannenlassen der Gummibirne 34 betätigbar. Mit ihr kann atmosphärische Luft, welche über das Ventil 36 angesaugt wird, durch das Ventil 30 hindurch in das Kissen 14 gedrückt werden, um dieses aufzublähen. Das Ventil 30 ist als Doppelrückschlagventil mit zwei Ventilgliedern 38 und 40 ausgestattet, deren eines, 38, von außen her über einen Druckknopf 42, der ebenfalls durch die Kleidung hindurch betätigbar ist, in seine Freigabestellung gebracht werden kann, um das Kissen 14 in die Atmosphäre zu entlüften. Gewünschtenfalls kann auch das vorausgehend erwähnte Druckbegrenzungsventil, wie etwa 25, in das Ventil 30 integriert sein.

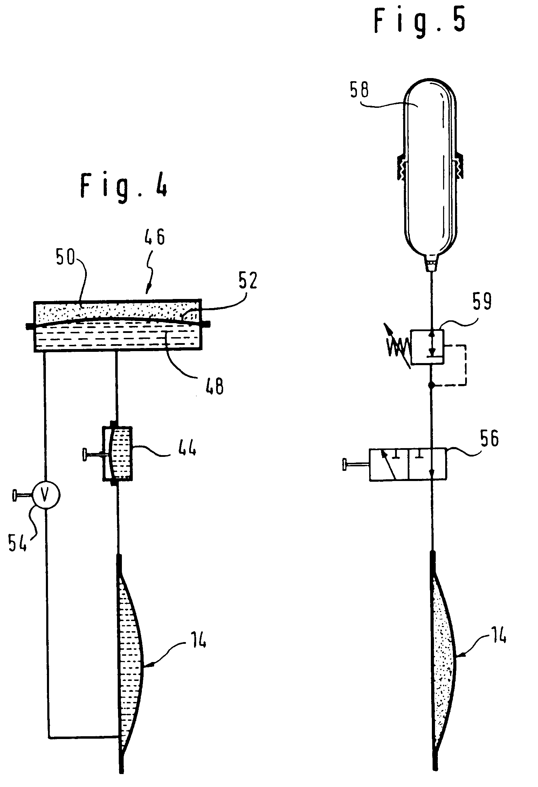

Fig. 4 zeigt rein schematisch eine alternative Ausführungsform, bei der Ventil 30 und Luftpumpe 32 aus den Figuren 1 und 3 durch eine Membranpumpe 44 in Verbindung mit einem Vorratsbehälter 46 ersetzt sind und aus dem Vorratsbehälter eine Flüssigkeit 48 in das Kissen, wie etwa 14, pumpbar ist. Zu diesem Zweck steht die Flüssigkeit 48 in dem Vorratsbehälter 46 unter dem Druck eines Gaspolsters 50, das durch eine Membran 52 von der Flüssigkeit 48 abgetrennt sein kann. Ein manuell betätigbares Rückflußventil 54 ermöglicht es, das Kissen 14 in den Vorratsbehälter 46 zurück zu entleeren. Der dazu erforderliche Druck auf das Kissen 14 kann vom Stumpf, etwa durch seitliches Ankippen der Prothese, aufgebracht werden. Anderenfalls ist es auch denkbar, zur Entleerung des Kissens eine zweite Membranpumpe oder dergl. vorzusehen. Im übrigen aber könnte der Vorratsbehälter 46 auch gleichfalls als Kissen ausgebildet werden, aus dem heraus durch manuellen Druck die Flüssigkeit 48 über ein manuell betägbares Absperrventil in das Kissen 14 verdrängt werden kann wie umgekehrt.

Wo, wie nach Fig. 1 und 2, innerhalb eines Prothesenschaftes mehrere Kissen, wie z.B. die Kissen 12, 14 und 20, vorgesehen sind, können diese selbstredend über entsprechend viele Ventile, wie z.B. 30 bzw. 54, mit einem einzigen Vorratsbehälter in Verbindung stehen.

An die Stelle manuell betätigbarer Pumpen, wie z.B. 32 und 44, können gewünschtenfalls natürlich auch elektrisch betätigbare, etwa aus einer mitgeführten Batterie speisbare Pumpen treten. Schließlich aber kann ein jedes der Kissen, wie z.B. 12, 14 und 20, gemäß Fig. 5 auch ohne Pumpen, nämlich durch ein manuell betätigbares Zweiwegeventil 56 hindurch mit einem gasförmigen Medium, vorzugsweise Luft, aus einem unter Druck stehenden Vorratsbehälter 58 heraus, aufgeblasen bzw. ins Freie entlüftet werden. Der Vorratsbehälter 58 ist dabei zweckmäßigerweise, wie gezeigt, als auswechselbare Patrone ausgebildet. Zwischen Vorratsbehälter 58 und Zweiwegeventil 56 ist ein Druckbegrenzungsventil in Gestalt eines Druckminderventils (Reduzierventils) 59 angeordnet.

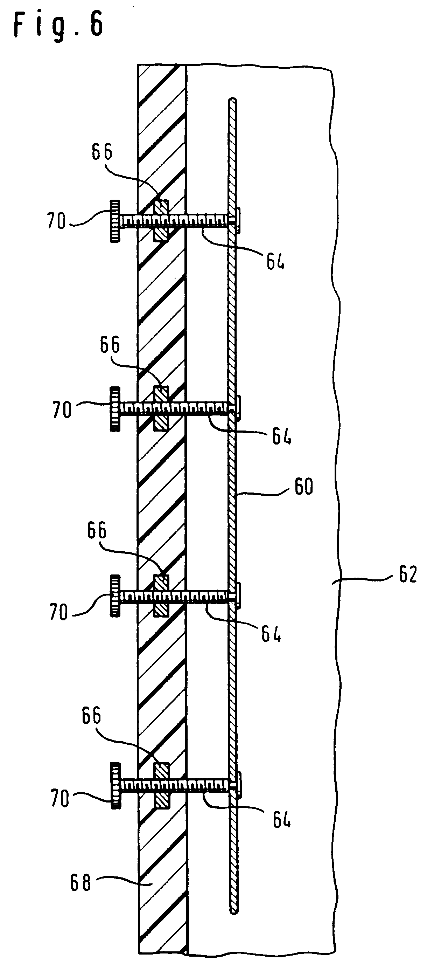

Fig. 6 gibt schließlich noch eine andere Ausführungsform für eine der seitlichen Backen im Prothesenschaft an. Die betreffende Backe wird von einer Federblechplatte 60 gebildet, die im Inneren des Schaftes 62 der Prothese, in diesem Falle eines einteiligen Schaftes, mittels mehrerer Schrauben 64 gehalten und abgestützt wird. Die Schrauben 64 sind in der Federblechplatte 60 drehbar verankert und durch Muttern 66 hindurchgeführt, die in die Wandung 68 des Schaftes 62 eingelassen sind. Zu ihrer individuellen Verstellung weisen die Schrauben 64 außerhalb der Wandung 68 Rändelköpfe 70 auf.

## Patentansprüche

1. Beinprothese mit schalenförmigem Schaft (4, 6), mindestens einer seitlich an der Schaftwandung (18) angeordneten Backe (16; 60) sowie einem im Stumpfendbereich angeordneten aufblähbaren Kissen (20), dadurch ***gekennzeichnet***, daß der Schaft (4, 6) im Stumpfendbereich im wesentlichen geschlossen und dem Stumpfende angepaßt ist und daß das dortige Kissen (20) auf der betreffenden Bodenpartie des Schaftes aufliegt und zumindest schaftinnenseitig aus einem Material besteht, welches weich genug ist, um sich an das Stumpfende anzuschmiegen.

2. Beinprothese nach Ansspruch 1, dadurch ***gekennzeichnet***, daß der Schaft in an sich bekannter Weise aus einem Außenschaft (4) und einem darin eingesetzten, den Konturen des betreffenden Gliedmaßenstumpfes genauer angepaßten Innenschaft (6) besteht und daß das im Stumpfendbereich auftretende Kissen (20) innerhalb des Innenschaftes (6) angeordnet ist.

3. Beinprothese nach Anspruch 2, dadurch ***gekennzeichnet***, daß der Innenschaft (6) zumindest in seinem Umfangsbereich flexibel ist und dort auftretende Backen (16) zwischen Außenschaft (4) und Innenschaft (6) angeordnet sind.

4. Beinprothese nach einem der vorhergehenden Ansprüche als Oberschenkelprothese, dadurch ***gekennzeichnet***, daß sie eine seitliche Backe (16; 60) von länglicher Form im dorso-lateralen Bereich im Anschluß an die Kante (22) des Oberschenkelknochens (24), sich von proximal nach distal erstreckend, und eine weitere, in annähernd runder Form, im medial-distalen Bereich aufweist.

5. Beinprothese nach einem der vorhergehenden Ansprüche, dadurch ***gekennzeichnet***, daß auch die seitlichen Backen (16) von aufblähbaren Kissen (12, 14) gebildet werden.

6. Beinprothese nach einem der vorhergehenden Ansprüche, dadurch ***gekennzeichnet***, daß das bzw. die Kissen (12, 14, 20) über ein Ventil (30; 56) aufblasbar bzw. aufpumpbar ist bzw. sind.

7. Beinprothese nach einem der Ansprüche 1 bis 5, dadurch ***gekennzeichnet***, daß das bzw. die Kissen (12, 14, 20) mit einem Fluid aus einem Vorratsbehälter (46; 58) befüllbar ist bzw. sind.

8. Beinprothese nach einem der vorhergehenden Ansprüche, dadurch ***gekennzeichnet***, daß das bzw. die Kissen (12, 14, 20) mit einem Druckbegrenzungsventil, wie z.B. einem Überdruckventil (25) oder einem Druckminderventil (59), in Verbindung steht bzw. stehen.

9. Beinprothese nach einem der vorhergehenden Ansprüche, dadurch ***gekennzeichnet***, daß zumindest die schaftinnenseitige Wand (16) des bzw. der Kissen (12, 14, 20) aus einem verhältnismäßig weichen, elastisch dehnfähigen Material wie z.B. Gummi oder elastomerem Polyurethan, kaschiert z.B. mit Textilien oder unkaschiert, besteht.

## Claims

1. Leg prosthesis with a shell-type shaft (4, 6), at least one cheek (16; 60) arranged laterally on the shaft wall (18), and an inflatable cushion (20) arranged in the stump end region, characterized in that the shaft (4, 6) is essentially closed in the stump end region and adapted to the stump end, and in that the cushion (20) there lies on the respective bottom portion of the shaft and, at least on the inner side of the shaft, consists of a material which is soft enough to mould itself snugly onto the stump end.

2. Leg prosthesis according to Claim 1, characterized in that the shaft consists, in a manner known per se, of an outer shaft (4) and of an inner shaft (6) fitted into the latter and adapted more exactly to the contours of the limb stump in question, and in that the cushion (20) appearing in the stump end region is arranged inside the inner shaft (6).

3. Leg prosthesis according to Claim 2, characterized in that the inner shaft (6) is flexible, at least in its circumferential area, and cheeks (16) appearing there are arranged between outer shaft (4) and inner shaft (6).

4. Leg prosthesis according to one of the preceding claims as a thigh prosthesis, characterized in that it includes a lateral cheek (16; 60) of elongate shape in the dorsolateral region adjacent the edge (22) of the femur (24), extending from proximal to distal, and a further one, of approximately round shape, in the medial-distal region.

5. Leg prosthesis according to one of the preceding claims, characterized in that the lateral cheeks (16) are also formed by inflatable cushions (12, 14).

6. Leg prosthesis according to one of the preceding claims, characterized in that the cushion or cushions (12, 14, 20) can be inflated or pumped up via a valve (30; 56).

7. Leg prosthesis according to one of Claims 1 to 5, characterized in that the cushion or cushions (12, 14, 20) can be filled with a fluid from a storage container (46; 58).

8. Leg prosthesis according to one of the preceding claims, characterized in that the cushion or cushions (12, 14, 20) is/are in communication with a pressure-limiting valve such as, for example, a pressure relief valve (25) or a pressure-reducing valve (59).

9. Leg prosthesis according to one of the preceding claims, characterized in that at least the wall (16) on the shaft inner side of the cushion or cushions (12, 14, 20) consists of a relatively soft, elastically extensible material such as, for example, rubber or elastomeric polyurethane, lined, for example, with textiles, or unlined.

## Revendications

1. Prothèse de jambe comportant une tige enveloppante (4, 6), au moins une mâchoire (16 ; 60) disposée latéralement sur la paroi de tige (18), et un coussin gonflable (20) disposé dans la région terminale du moignon,
caractérisée en ce que la tige (4, 6) est sensiblement fermée dans la région terminale du moignon et adaptée à l'extrémité du moignon, et en ce que le coussin (20) qui y est disposé repose sur la portion de fond de la tige, et est réalisé, au moins du côté en regard de l'intérieur de tige, en une matière assez tendre pour épouser la forme de l'extrémité du moignon.

2. Prothèse de jambe selon la revendication 1,
caractérisée en ce que la tige consiste, de manière connue en soi, en une tige externe (4) et une tige interne (6) qui y est insérée et qui est adaptée de manière plus précise au contour du moignon de membre, et en ce que le coussin (20) placé dans la région terminale du moignon est disposé à l'intérieur de la tige interne (6).

3. Prothèse de jambe selon la revendication 2,
caractérisée en ce que la tige interne (6) est souple, au moins dans sa région périphérique, et en ce que les mâchoires (16) qui y sont placées sont disposées entre la tige externe (4) et la tige interne (6).

4. Prothèse de jambe selon l'une des revendications précédentes, en forme de prothèse fémorale,
caractérisée en ce qu'elle comporte une mâchoire latérale (16 ; 60) de forme allongée, située dans la région dorso-latérale, dans le prolongement de l'arête (22) de l'os fémoral (24), et s'étendant de la région proximale vers la région distale, ainsi qu'une autre mâchoire, sensiblement ronde, située dans la région médiane-distale.

5. Prothèse de jambe selon l'une des revendications précédentes,
caractérisée en ce que les mâchoires latérales (16) sont aussi formées par des coussins gonflables (12, 14).

6. Prothèse de jambe selon l'une des revendications précédentes,
caractérisée en ce que le coussin ou les coussins (12, 14, 20) est ou sont gonflable(s), éventuellement à l'aide d'une pompe, au travers d'une valve (30 ; 56).

7. Prothèse de jambe selon l'une des revendications 1 à 5,
caractérisée en ce que le coussin ou les coussins (12, 14, 20) peut ou peuvent être rempli(s) d'un liquide venant d'un réservoir (46 ; 58).

8. Prothèse de jambe selon l'une des revendications précédentes,
caractérisée en ce que le coussin ou les coussins (12, 14, 20) est ou sont en relation avec une soupape de limitation de la pression, telle que, par exemple, une soupape de surpression (25) ou une vanne de détente (59).

9. Prothèse de jambe selon l'une des revendications précédentes,
caractérisée en ce que au moins la paroi (16) située vers l'intérieur de la tige, du coussin ou des coussins (12, 14, 20) est ou sont réalisée(s) en une matière relativement tendre, élastiquement extensible, telle que, par exemple le caoutchouc ou un polyuréthane élastomère, revêtue par exemple d'un textile ou qui est à nu.
